# EUROPEAN PATENT APPLICATION

(11) **EP 4 238 553 A1**
(43) Date of publication of application: **06.09.2023**
(21) Application number: 22305231.7
(22) Date of filing: 01.03.2022
(51) Int. Cl.: A61K 9/00, A61K 9/08

(54) **COMPOSITIONS AND METHODS FOR DELIVERING CLOFOCTOL**

(71) Applicant: Apteeus, 59000 Lille (FR)
(72) Inventor: BEGHYN, Terence, 59320 HAUBOURDIN (FR); MOREAU VANBÉSIEN, Camille, 59280 ARMENTIÈRES (FR); BELLOY, Loïc, 59263 HOUPLIN-ANCOISNE (FR)
(74) Representative: Cabinet Beau de Loménie

(57) **Abstract**

The present invention relates to an aerosol containing clofoctol, wherein the aerosol particles have a mean diameter D(v;0.5) of between about 0.25 and about 5 microns and contain at most 15.0% by weight of clofoctol. The invention also relates to a composition suitable for generating said aerosol, and to the use of the aerosol in therapy.

## Description

### FIELD OF THE INVENTION

The present invention relates to compositions and methods for delivering clofoctol through an inhalation route.

### BACKGROUND OF THE INVENTION

Gramplus^{®} is a composition currently marketed for the treatment of respiratory infection due to bacteria (ref. 1). It is administered rectally as suppositories. The active ingredient is clofoctol which is typically provided in doses of 100, 200 and 750mg. Clofoctol has recently been reported to be effective against coronavirus infections (ref. 2), and to display an anti-inflammatory activity (ref. 3). It has also been described to prevent lysosphingolipid-induced lysosomal impairment (ref. 4). Clofoctol is also known to reduce prostate cancer growth via synergistic induction of endoplasmic reticulum stress and UPR pathways (ref. 5). The current route of administration of clofoctol has a number of limitations. Rectal administration is not convenient in every situation: diarrhea can prevent the administration; the suppository can be ejected after administration without the possibility to re-administer it. Despite a local site of action, the whole organism is exposed to the drug. It is therefore desirable to provide a new route of administration for clofoctol that allows for a rapid onset of action without the disadvantages of rectal administration. While inhalation has long been established as an effective way to deliver drugs to the respiratory airways, there are limiting factors concerning the administration of clofoctol by such a route. On the one hand, the low melting point (78°C) of clofoctol is limiting the possibilities to prepare formulation dedicated to pressurized metered-dose inhalers and dry powder inhalers. On the other hand, the very low aqueous solubility of clofoctol is limiting the use of nebulizers. It is therefore desirable to provide a new composition and method for delivering clofoctol through the inhalation route. The provision of such a composition and method is an object of the present invention.

### SUMMARY OF THE INVENTION

The present invention relates to compositions and methods for delivering clofoctol through an inhalation route. Specifically, it relates to aerosols containing clofoctol that can be used in inhalation therapy, and to compositions suitable for generating such aerosols. In one aspect, the present invention relates to an aerosol containing clofoctol, wherein the aerosol particles have a mean diameter D(v;0.5) of between about 0.25 and about 5 microns and contain at most 15.0% by weight of clofoctol.

In some embodiments the aerosol has an aerosol drug mass density of between about 0.01 mg/L and about 1 mg/L. In some embodiments, the aerosol has a volume concentration of less than about 0.05%.

In one aspect, the present invention relates to a composition suitable for generating the aerosol defined above, comprising, by weight of the composition:
a) 95.0 to 99.9% of propylene glycol or of a mixture of propylene glycol and glycerin;
b) 0.1 to 5.0% of clofoctol.

In some embodiments, component a) is propylene glycol. In some embodiments component a) is a mixture of propylene glycol and glycerin.

In one aspect, the present invention relates to a method of producing an aerosol comprising:
a) heating the composition defined above to vaporize it;
b) passing air through the vaporized composition to produce the aerosol.

In one aspect the present invention relates to a method of treating a subject in need of clofoctol treatment which comprises administering the aerosol defined above to the subject.

In one aspect, the present invention relates to the aerosol defined above for use in a method of treatment of a disease where clofoctol treatment is indicated. In some embodiments, the disease is a disease caused by a coronavirus, an infection caused by bacteria, or an inflammatory disease.

### BRIEF DESCRIPTIO OF THE FIGURES

Figure 1 represents clofoctol plasma concentrations as a function of time in mice inhaling an aerosol according to the invention.
Figure 2 represents clofoctol lung concentrations as a function of time in mice inhaling an aerosol according to the invention.

### DETAILED DESCRIPTION OF THE INVENTION

### Definitions

The term "aerosol" refers to a suspension of fine solid particles or liquid droplets in a gas. The expression "(aerosol) drug mass density" refers to the mass of clofoctol per unit volume of aerosol. It is calculated by measuring the quantity of clofoctol in a determined volume of aerosol beforehand trapped.

"Clofoctol" is 2-[(2,4-dichlorophenyl)methyl]-4-(2,4,4-trimethylpentan-2-yl)phenol (CAS 37693-01-9).

The "median diameter" (D(v;0.5)) of an aerosol refers to the diameter for which half the particulate mass of the aerosol consists of particles with a diameter larger than the median diameter and half by particles with a diameter smaller than the median diameter. The median diameter is measured by laser light scattering using for example a Malvern Mastersizer X or any equivalent apparatus working on the same principle of laser light scattering.

The "span" of a volume-based size distribution is defined by Span = (D(v;0.9) - D(v;0.1)) / D(v;0.5) and gives an indication of how far the 10 percent and 90 percent points are apart, normalized by the midpoint.

A "Thermal Aerosol Generator" or "TAG" is a device which produces inhalable aerosols by heating a liquid with an electrically powered metallic/ceramic coil.

The "volume concentration" is the volume of particles reported to the total volume of the aerosol expressed as a percentage. The volume concentration is measured by laser light scattering using for example a Malvern Mastersizer X or any equivalent apparatus working on the same principle of laser light scattering.

The expression "between x and y" shall be understood as including the end points of the range considered (i.e., x and y).

In the context of the present disclosure, the embodiments described herein can be combined one with the other.

In one aspect, the present invention relates to an aerosol containing clofoctol, wherein the aerosol particles have a mean diameter D(v;0.5) of between about 0.25 and about 5 microns and contain at most 15.0% by weight of clofoctol.

In some embodiments, the aerosol particles have a mean diameter D(v;0.5) of between about 1 and about 3 microns.

In some embodiments, the span of size distribution is less than about 3.0, such as for example less than about 1.0, and is advantageously of about 0.5.

In some embodiments the aerosol particles comprise at most 10.0% by weight, such as for example at most 5.0% by weight, at most 2.0% by weight, at most 1.0% by weight or at most 0.1% by weight of clofoctol.

In some embodiments the aerosol has an aerosol drug mass density of between about 0.01 mg/L and about 1 mg/L.

In some embodiments, the aerosol has a volume concentration of less than about 0.05%, such as for example between about 0.001 and about 0.01%, or between about 0.001 and about 0.005%.

In some embodiments, the aerosol has an (aerosol) drug mass density of between about 0.01 mg/L and about 1 mg/L, such as for example between about 0.05 mg/L and about 0.5 mg/L, or between about 0.05 mg/L and about 0.3 mg/L.

In some embodiments, the aerosol particles comprise clofoctol having a purity of more than about 80.0%, such as for example more than about 95.0%, more than about 97.5%, more than about 99.0%, more than about 99.5%, more than about 99.9% or more than 99.97%.

In one aspect, the present invention relates to a composition for generating an aerosol as defined herein.

In some embodiments, the composition comprises, by weight of the composition:
a) about 95.0 to about 99.9% of propylene glycol or of a mixture of propylene glycol and glycerin;
b) about 0.1 to about 5.0% of clofoctol.

In some embodiments, the composition comprises:
a) about 97.0 to about 99.5%, by weight of the composition, of propylene glycol or of a mixture of propylene glycol and glycerin;
b) about 0.5 to about 3.0%, by weight of the composition, of clofoctol.

In some embodiments, component a) is propylene glycol.

In some embodiments, component a) is mixture of propylene glycol and glycerin in a weight ratio of about 95/5 to about 50/50, such as for example from about 90/10 to about 70/30.

In some embodiments, the composition may further comprise up to about 5.0% by weight, based on the combined weight of a) and b), of one or more flavouring agents. Examples of suitable flavouring agents include menthol, licorice, chocolate, fruit flavour (including, e.g., citrus, cherry, etc.), vanilla, spice (e.g., ginger, cinnamon) and tobacco flavour.

In embodiments where the aerosol is generated from the composition containing a) and b) as defined herein, the aerosol particles advantageously comprise at least about 50.0% by weight of propylene glycol, such as for example at least about 60.0% by weight, at least about 70.0% by weight, at least 80.0% by weight, at least about 90.0% by weight or at least about 95.0% by weight of propylene glycol.

In one aspect, the present invention relates to a method for obtaining an aerosol as defined herein.

In some embodiments, the method comprises:
a) heating a composition as defined herein to vaporize it, and
b) passing air through the vaporized composition to obtain the aerosol.

Any suitable method can be used to heat the composition. Examples of methods by which heat can be generated include the following: passage of current through an electrical resistance element; absorption of electromagnetic radiation, such as microwave or laser light; and exothermic chemical reactions, such as exothermic salvation, hydration of pyrophoric materials and oxidation of combustible materials. Typically, the composition is heated on an electrically-powered heating element such as a metallic or ceramic coil. When the composition is heated, propylene glycol and glycerin (when present) are vaporized thereby generating droplets which carry away (or "trap") clofoctol.

In one aspect the present invention relates to a method of treating a subject in need of clofoctol treatment which comprises administering the aerosol defined above to the subject.

In some embodiments, the subject in need of clofoctol treatment is a subject suffering from: a disease caused by a coronavirus; a bacterial, viral or fungal infection of upper and lower airways; or an inflammatory disease.

Diseases caused by a coronavirus include diseases caused by SARS-CoV, MERS-CoV, SARS-CoV-2, HCoV-NL63, HCoV-OC43, HCoV-HKU1 or HCoV-229E. Clofoctol is particularly suitable for treatment of a disease caused by a coronavirus such as SARS-CoV, MERS-CoV, SARS-CoV-2 and variants thereof.

Inflammatory diseases include clinical disorders in which inflammation is a prominent contributor to the clinical condition. Typically, the inflammatory disease is selected from the group consisting of asthma, chronic obstructive lung disease, pulmonary fibrosis, pneumonitis (including hypersensitivity pneumonitis and radiation pneumonitis), pneumonia, cystic fibrosis, psoriasis, arthritis/rheumatoid arthritis, rhinitis, pharyngitis, cystitis, prostatitis, dermatitis, allergy including hay fever, nephritis, conjunctivitis, encephalitis, meningitis, ophthalmitis, uveitis, pleuritis, pericarditis, myocarditis, atherosclerosis, human immunodeficiency virus related inflammation, diabetes, osteoarthritis, psoriatic arthritis, inflammatory bowel disease (Crohn's disease, ulcerative colitis)/colitis, sepsis, vasculitis, bursitis, connective tissue disease, autoimmune diseases such as systemic lupus erythematosis (SLE), polymyalgia rheumatica, scleroderma, Wegener's granulomatosis, temporal arteritis, vasculitis, cryoglobulinemia, and multiple sclerosis.

Inflammatory diseases also include an inflammation secondary to infection caused by virus, bacteria, fungi and parasites.

In some embodiments the aerosol is delivered to the subject using a thermal aerosol generator. Typically, this device comprises at least: means for heating the clofoctol-containing composition; means for providing an airflow allowing the generation of the aerosol; and means allowing the subject to inhale the aerosol. Various suitable heating methods are described above. The means for providing an airflow is, in its simplest form, an inert passageway linking the heating means to the inhalation means. The means permitting inhalation is an aerosol exit portal that forms a connection between the airflow ways and the subject's respiratory system.

In some embodiments, devices used to deliver the aerosol also include any component known in the art to control the timing of drug aerosolization relative to inhalation, to provide feedback to patients on the rate and/or volume of inhalation, to prevent excessive use, to prevent use by unauthorized individuals, and/or to record dosing histories.

In one aspect, the present invention relates to the aerosol defined above for use in a method of treatment of a disease where clofoctol treatment is indicated. In some embodiments, the disease is as defined above.

In some embodiments, the aerosol is either administered as a single inhalation or as a series of inhalations taken within an hour or less. Where the drug is administered as a series of inhalations, a different amount may be delivered in each inhalation.

The following examples are meant to illustrate, rather than limit, the present invention. In these examples, the following abbreviations are used.
CFT = clofoctol
Conc. = concentration
Gly = glycerin
PG = propylene glycol
RT = room temperature
TAG = thermal aerosol generator

### Examples

### Analysis of clofoctol-containinq aerosols

The purity of a clofoctol-containing aerosol, and quantitation of clofoctol in this aerosol was determined using a method that involves forming the aerosol in a device through which an air flow is maintained, generally at a rate between 1 and 180 L/min. The gas flow carries the aerosol into one or more traps. After extraction from the trap, the trapped components from the aerosol are subjected to an analytical technique, such as liquid chromatography, that permits a determination of composition and purity. The liquid chromatography discussed above contains a detection system (i.e., detector). Such detection systems are well known in the art and include, for example, UV detectors and mass spectrometry detectors. Liquid chromatography-mass spectrometry is the preferred analytical technique.

### Example 1: solubility of clofoctol

Solutions of clofoctol were prepared in different solvents: 100% PG, 85/15 PG/Gly, 70/30 PG/Gly, 50/50 PG/Gly. Solutions were sonicated then incubated at 65°C for 30 min, until complete dissolution of clofoctol. Then, the samples were incubated at RT under stirring for different times. For collection before analysis after determined incubation times, the solutions were centrifuged, then the supernatants were collected and diluted before LC-MS/MS analysis for clofoctol quantitation in samples.

The results are presented in Table 1.

**Table 1**

| | After 48h incubation | | After > 120h incubation | |
|---|---|---|---|---|
| **Solvent composition** | **CFT conc. in collected sample (mM)** | **CFT conc. in collected sample (mg/g)** | **CFT conc. in collected sample (mM)** | **CFT conc. in collected sample (mg/g)** |
| 50/50 PG/Gly | 7.9 | 2.5 | 6.7 | 2.1 |
| 70/30 PG/Gly | 29.8 | 9.8 | 28.1 | 9.2 |
| 85/15 PG/Gly | 503.5 | 171.9 | 146.7 | 50.1 |
| 100% PG | 489.0 | 171.8 | 441.0 | 154.9 |

It can be seen from this table that the maximum concentration achieved when the clofoctol solutions are stored under stirring for more than 5 days, is about 15% by weight (154.9 mg/g).

### Example 2: aerosol drug mass density

Solutions containing 20 mg/g of clofoctol in various solvents were prepared. Aerosols were produced using a TAG (Vaporesso swag 2 with a coil resistance of 0.3 ohms) at different powers and an airflow of 34 L per minute. 1.416 L of emitted aerosol was trapped on a plastic filter. Clofoctol was then extracted from the filter and dosed by LC-MS to determine the drug mass density. The results are presented in Table 2.

**Table 2**

| **Solvent composition** | **Power (W)** | **Collected mass of clofoctol in the trap (µg)** | **Drug mass density (mg/L)** |
|---|---|---|---|
| 100% PG | 30 | 225.6 | 0.159 |
| 85/15PG/Gly | 30 | 135.8 | 0.096 |
| | 50 | 141.1 | 0.099 |
| | 80 | 100.5 | 0.071 |
| 70/30 PG/Gly | 30 | 128.3 | 0.091 |

### Example 3: characterization of aerosol particles

Solutions containing 10 mg/g or 20 mg/g of clofoctol in various solvents were prepared. Aerosols were produced using a TAG (Vaporesso swag 2 with a coil resistance of 0.3 ohms) at different powers. The flow of aerosol was generated using an air flow of 20L/min. A certain volume of emitted aerosol was passed through the laser beam of a granulometer, Mastersizer X from Malvern. The results are presented in Table 3.

**Table 3**

| **Solvent composition** | **CFT conc. (mg/g)** | **Power (W)** | **Mean diameter (µm)** | **Median diameter (µm)** | **Sauter diameter (µm)** | **Span** | **Volume conc. (%)** |
|---|---|---|---|---|---|---|---|
| 100% PG | 20 | 30 | 2.78 | 2.75 | 2.66 | 0.521 | 0.0038 |
| 70/30 PG/Gly | 20 | 30 | 2.89 | 2.87 | 2.76 | 0.551 | 0.0020 |
| 85/15 PG/Gly | 10 | 30 | 2.45 | 2.42 | 2.34 | 0.519 | 0.0052 |
| | 10 | 45 | 2.40 | 2.38 | 2.30 | 0.523 | 0.0027 |
| | 10 | 60 | 2.63 | 2.59 | 2.46 | 0.635 | 0.0018 |
| | 20 | 30 | 2.41 | 2.38 | 2.30 | 0.543 | 0.0016 |

### Example 4: determination of clofoctol purity in the aerosol

Solutions containing 20 mg/g of clofoctol in various solvents were prepared. Aerosols were produced using a TAG (Vaporesso swag 2 with a coil resistance of 0.3 ohms) at different powers. The flow of aerosol was generated using an air flow of 20 L/min. One or two successive puffs of 2.5s each was (were) done for each condition. For each puff, a certain volume of emitted aerosol was trapped on a plastic filter. Clofoctol was then extracted from the filter and analysed by LC-UV-MS. The results are presented in Table 4.

**Table 4**

| **Solvent composition** | **Power (W)** | **Puff number** | **Purity CFT (%)** | **Mean purity (%)** |
|---|---|---|---|---|
| 100% PG | 30 | 1 | 100.0 | 100.0 |
| | | 2 | 100.0 | |
| | 80 | 1 | 92.0 | 87.4 |
| | | 2 | 82.7 | |
| 70/30 PG/Gly | 30 | 1 | 100.0 | 100.0 |
| | | 2 | 100.0 | |
| | 80 | 1 | 91.4 | 91.0 |
| | | 2 | 90.6 | |

### Example 5: determination of drug exposure in mice after inhalation

Mice (Charles River, Female CD-1, 20-23 g) were permitted water and food ad libitum during the experiment. Clofoctol was administrated by Nose-Only inhalation in a homemade inhalation chamber in part inspired by a previous described apparatus (ref. 6). Animals were acclimated to restraint during a week before the experiment started. Animals were exposed to an aerosol generated (using a TAG connected to the chamber: Vaporesso swag 2 with a coil resistance of 0.3 ohms) from a solution of 20 mg/g of clofoctol (Polyethylene Glycol/Glycerin, 85/15) for a period of 15 min with a puff of 4.5 s every minute (with a power of 30W).

Blood and lungs (left lung) were sampled 5, 10, 15, 30, 60 and 120 minutes after the end of a single inhalation period (2 mice/sampling time). The blood was sampled in tubes with Lithium Heparin on ice and then the plasma was separated from the red cells by centrifugation and frozen (-80°C).

Clofoctol was extracted from plasma by protein precipitation at 1/10th with ethanol. The samples were vortexed, then centrifuged for 10 min at 6,000 rpm, at room temperature. The supernatants were transferred into Matrix tubes for LC-MS/MS analysis.

### Clofoctol plasma concentrations

The unknown plasma concentrations were calculated from a calibration curve prepared by spiking clofoctol in commercial mouse plasma (Male Mouse LIHP CD-1 MSEPLLIHP-M, Seralab) and reprocessed in the same way as the samples (extraction in 1/10th ethanol).

The analysis was conducted on an Acquity I-Class UPLC (Waters), coupled to a Xevo TQS micro triple quadrupole (Waters) with an ESI source, and operating in SIR and pseudo-MRM modes, with the following parameters. The separation was done using a BEH C8 1.7µm 2.1x50mm column, kept at 40°C, and eluents at 5mM ammonium formate, pH 3.8, in water (A1) and water/acetonitrile 5/95 (B1). The flow rate was set at 0.7mL/min.

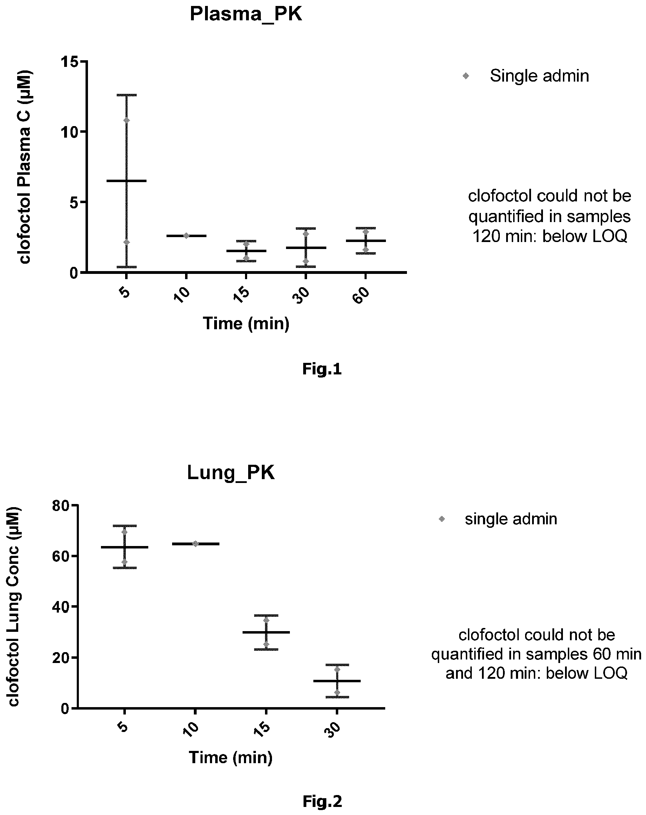

The results are shown in Figure 1.

### Clofoctol lung concentrations

A known mass (approximately 15mg, weighed beforehand) of each lung was placed into a 'safe lock' microtube (2 mL). Clofoctol was extracted by adding 1000µL of ethanol as well as a ball of tungsten carbide (5 mm) to the tube, and samples were homogenized using Tissue Lyzer II (from Qiagen), for 2x5s at 25Hz. Lung homogenates obtained were centrifuged for 10 min at 6,000 rpm, at room temperature. The supernatants were transferred into Matrix tubes for LC-MS/MS analysis, with the same LC and MS/MS parameters as for the plasma analysis.

The unknown lung concentrations of clofoctol were calculated from a calibration line produced in the same matrix (naive lung from 'vehicle' animals) and reprocessed in the same way as the samples (homogenization in ethanol).

The results are shown in Figure 2.

It can be seen from Figures 1 and 2 that lungs from mice were very well exposed to clofoctol with a maximum Cmax (Cmax = 64.8µM) obtained within 5 minutes from administration, while the plasma concentration remained low (Cmax = 6.5µM).

### References

1. R. Danesi et M. Del Tacca, « Clinical study on the efficacy of clofoctol in the treatment of infectious respiratory diseases. », Int J Clin Pharmacol Res, vol. 5, n° 3, p. 175-179, 1985.
2. International application PCT/EP2021/065356
3. European patent application n° 21 305 619.5 filed on 12 May 2021
4. C. J. Folts et al., « Lysosomal Re-acidification Prevents Lysosphingolipid-Induced Lysosomal Impairment and Cellular Toxicity », PLoS Biol, vol. 14, n° 12, p. e1002583, dec. 2016, doi: 10.1371/journal.pbio. 1002583.
5. L. Fan et al., « Clofoctol and sorafenib inhibit prostate cancer growth via synergistic induction of endoplasmic reticulum stress and UPR pathways », CMAR, vol. Volume 10, p. 4817-4829, oct. 2018, doi: 10.2147/CMAR.S175256.
6. R. Kaur et al., « An Efficient and Cost-Effective Nose-Only Inhalational Chamber for Rodents: Design, Optimization and Validation », AAPS PharmSciTech 21, 82 (2020).

## Claims

1. An aerosol containing clofoctol, wherein the aerosol particles have a mean diameter D(v;0.5) of between about 0.25 and about 5 microns and contain at most 15.0% by weight of clofoctol.

2. The aerosol of claim 1, wherein the aerosol particles have a mean diameter D(v;0.5) of between about 1 and about 3 microns.

3. The aerosol of claim 1 or claim 2, wherein the aerosol particles comprise at most 10.0% by weight, such as at most 5.0% by weight, at most 2.0% by weight, at most 1.0% by weight or at most 0.1% by weight of clofoctol.

4. The aerosol of any preceding claims, which has an aerosol drug mass density of between about 0.01 mg/L and about 1 mg/L.

5. The aerosol of any preceding claims, which has a volume concentration of less than about 0.05%%.

6. A composition for generating an aerosol as defined in any of the preceding claims, comprising, based on the weight of the composition:
a) about 95.0 to about 99.9% of propylene glycol or of a mixture of propylene glycol and glycerin;
b) 0.1 to 5.0 % of clofoctol.

7. The composition of claim 6, comprising:
a) about 97.0 to about 99.5% of propylene glycol or a mixture of propylene glycol and glycerin;
b) about 0.5 to about 3.0 % of clofoctol.

8. The composition of claim 6 or claim 7, wherein component a) is propylene glycol.

9. The composition of claim 6 or claim 7, wherein component a) is a mixture of propylene glycol and glycerin in a weight ratio of about 99/1 to about 50/50, such as from about 90/10 to about 70/30.

10. A method for obtaining an aerosol as defined in any of claims 1 to 5, comprising:
a) heating a composition as defined in any of claims 6 to 9 to vaporize said composition, and
b) passing air through the vaporized composition to obtain the aerosol.

11. An aerosol as defined in any of claims 1 to 5 or as obtained from a composition as defined in any of claims 6 to 9, for use in a method of treatment of: a disease caused by a coronavirus; a bacterial, viral or fungal infection of upper and lower airways; or an inflammatory disease.

12. The aerosol for use of claim 11, for the treatment of an inflammatory disease.

13. A method of treating a subject in need of clofoctol treatment which comprises administering to the subject an aerosol as defined in any of claims 1 to 5 or as obtained from a composition as defined in any of claims 6 to 9.

14. The method of claim 13, wherein the subject in need of clofoctol treatment is a subject suffering from: a disease caused by a coronavirus; a bacterial, viral or fungal infection of upper and lower airways; or an inflammatory disease.
